# EUROPEAN PATENT SPECIFICATION

(11) **EP 1 719 520 B1**
(45) Date of publication and mention of the grant of the patent: **15.04.2015**
(21) Application number: 03756419.2
(22) Date of filing: 27.09.2003
(51) Int. Cl.: A61K 38/16, C07K 7/08, A61K 39/085, C07K 14/31, A61P 31/04

(54) **ANTIGEN EPITOPES OF THE REGULATORY PROTEIN OF VIRULENCE FACTOR IN STAPHYLOCOCCUS AUREUS AND THEIR MIMOTOPES AND USE**
ANTIGEN-EPITOPE DES REGULATOR-PROTEINS DES VIRULENZFAKTORS IN STAPHYLOCOCCUS AUREUS UND IHRE MIMOTOPE UND VERWENDUNG
EPITOPES ANTIGENIQUES DE LA PROTEINE DE REGULATION DE L'AGGRESSINE CHEZ LE STAPHYLOCOQUE DORE, LEURS MIMOTOPES ET LEUR UTILISATION

(30) Priority: 21.07.2003 CN 03150203
(43) Date of publication of application: 08.11.2006
(73) Proprietor: Applied NanoSystems B.V., 9700 AC Groningen (NL)
(72) Inventor: SHAO, Ningsheng, Haidian District, Beijing 100850 (CN); YANG, Guang, Haidian District, Beijing 100850 (CN); LIU, Chuan, Haidian District, Beijing 100850 (CN); GAO, Yaping, Haidian District, Beijing 100850 (CN); DONG, Jie, Haidian District, Beijing 100850 (CN); DING, Hongmei, Haidian District, Beijing 100850 (CN); SHEN, Beifen, Haidian District, Beijing 100850 (CN)
(74) Representative: Jansen, Cornelis Marinus
(86) International application number: PCT/CN2003/000827
(87) International publication number: WO 2005/007683

(56) References cited:
- WO-A-00/15660
- US-A1- 2002 102 271
- YANG GUANG ET AL: "A novel peptide screened by phage display can mimic TRAP antigen epitope against Staphylococcus aureus infections" JOURNAL OF BIOLOGICAL CHEMISTRY, vol. 280, no. 29, July 2005 (2005-07), pages 27431-27435, XP002527648 ISSN: 0021-9258
- WANG Y.: 'Phage Display Technology and its Appplication in research of antigenic epitope' CHINESE JOURNAL OF VETERINARY MEDICINE vol. 39, no. 4, April 2003, pages 34 - 37, XP008100491
- BALABAN N. ET AL: 'Regulation of Staphylococcus aureus Pathogenesis via Target of RNAIII-activatingProtein (TRAP).' JOURNAL OF BIOLOGICAL CHEMISTRY 26 January 2001, pages 2658 - 2667, XP002984048

### SEQUENCE LISTING

<110> Institute of Basic Medical Sciences Academy of Military
   Medical Sciences
   HAINAN GT-UNIPUT PHARMACEUTICAL CO. LTD.
<120> Antigen epitopes and mimotopes of TRAP (Target of RNA
   III activating protein), a regulatory protein of virulence factor in staphylococcus aureus and use
<130> P77792EP00
<140> EP 03756419.2
   <141> 2006-09-29
<150> CN 03150203.2
   <151> 2003-07-21
<150> PCT/CN2003/000827
   <151> 2003-09-27
<160> 5
<170> PatentIn version 3.3
<210> 1
   <211> 14
   <212> PRT
   <213> Staphylococcus aureus
<220>
   <221> DOMAIN
   <222> (1)..(14)
   <223> Corresponding to amino acids 21-34 of TRAP
<400> 1
   Asn Pro Thr His Gln Leu Phe Gln Phe Ser Ala Ser Asp Thr
   1               5                   10
<210> 2
   <211> 12
   <212> PRT
   <213> Staphylococcus aureus
<220>
   <221> DOMAIN
   <222> (1)..(12)
   <223> Corresponding to amino acids 156-167 of TRAP
<400> 2
   Ser Tyr Phe Glu Arg Tyr Leu Tyr Pro Ile Lys Glu
   1               5                   10
<210> 3
   <211> 12
   <212> PRT
   <213> Artificial
<220>
   <223> Antigen mimotope 1
<220>
   <221> misc_feature
   <222> (1)..(1)
   <223> Xaa can be any naturally occurring amino acid
<220>
   <221> misc_feature
   <222> (3)..(3)
   <223> Xaa can be any naturally occurring amino acid
<220>
   <221> misc_feature
   <222> (8)..(8)
   <223> Xaa can be any naturally occurring amino acid
<400> 3
   Xaa Pro Xaa His His Gln His Xaa Thr Gly Phe Thr
   1 5 10
<210> 4
   <211> 12
   <212> PRT
   <213> Artificial
<220>
   <223> Antigen mimotope 2
<220>
   <221> misc_feature
   <222> (5)..(6)
   <223> Xaa can be any naturally occurring amino acid
<220>
   <221> misc_feature
   <222> (10)..(12)
   <223> Xaa can be any naturally occurring amino acid
<400> 4
   Ser Trp Phe Asp Xaa Xaa Leu Tyr Pro Xaa Xaa Xaa
   1 5 10
<210> 5
   <211> 12
   <212> PRT
   <213> Artificial
<220>
   <223> Antigen mimotope sequence 1
<220>
   <221> DOMAIN
   <222> (1)..(12)
   <223> Similar to the sequence of amino acids 21-34 of TRAP
<400> 5
   Asn Pro Leu His His Glu His Ala Thr Gly Trp Thr
   1 5 10

## Description

### Technical Field

The present invention relates to antigen epitopes and mimotopes thereof, in particular to two antigen epitopes of TRAP (Target of RNA III-activating protein), a regulatory protein of virulence factor in *staphylococcus aureus* and their mimotopes. The present invention further relates to the use of polypeptides having amino acid sequences identical to these epitopes in the manufacture of a novel vaccine or medicament for combating against *staphylococcus aureus* infection.

### Background Art

*Staphylococcus aureus,* a common Gram-positive pathogenic bacteria, is a major microorganism that causes life threatening diseases such as infections of burn and war wound, pneumonia, endocarditis, septicemia, toxic shock, etc. There are more than millions of patients suffering with *staphylococcus aureus* infections only in hospital per year. At present, the combined administration of antibiotics is mainly used for clinical treatment of *staphylococcus aureus* infections, but its therapeutical effects are still unsatisfactory. Since *staphylococcus aureus* may readily develop drug resistance and no good solution exists, many common antibiotics are ineffective, so against it the control of *staphylococcus aureus* infection is one of the most urgent problems to be solved in clinical medicine.

The main pathogenic substances of *staphylococcus aureus* are toxins, including hematoxin, leucocidin, enterotoxin, etc. The recent researches indicate that the synthesis of these virulence factors in *staphylococcus aureus* is controlled by a regulatable RNA molecule and RNA III. RNA III activates the gene transcription of virulence factors, and regulates the translation of virulence factors by base complementarity. The RNA III level is relatively low during the early log phase of bacterial growth, but increases 40 times during the later log phase of bacterial growth, while the level of RNA III is regulated by the protein RAP (RNA III activating protein) secreted by *staphylococcus aureus* per se, so that RAP is also called as *staphylococcus aureus* virulence stimulating factor. *Staphylococcus aureus* continuously secrets RAP, and RAP activates the production of virulence factors only when RAP reaches a certain concentration. *Staphylococcus aureus* without the production of RAP does not cause diseases. The recent researches show that RAP activates the transcription of RNA III through the mediation of a 21 KD protein TRAP (Target of RNA III -activating protein). When the gene encoding TRAP is inactivated by mutation, RAP cannot activate the transcription of RNA III. TRAP consists of 167 amino acids and has His kinase activity. TRAP is phosphorylated during the early phase of the growth of *staphylococcus aureus,* and reaches the maximum level during the log growth metaphase. After the RAP action, the signal transduction is performed by autophosphorylation. It increases the intracellular level of RNA III , and accelerates the secretion of *staphylococcus aureus* exotoxins (Naomi B, et al, J. Biol, Chem, 2001, 276:2658-2667). It can be seen that TRAP plays a critical role in the regulation of toxin expression in *staphylococcus aureus.* The researches in 2001 indicate that antibodies against TRAP can effectively reduce the secretion of *staphylococcus aureus* exotoxins (Oleny V, et al., Peptides 2001, 22:1621-1627).

As target structures recognized by immune cells and substance basis for activating specific immune response, antigen epitopes are important for studies on immune responses. With the application of antigen deletion mutation, pepscan, X-ray crystal diffraction, etc., it is possible to determine "antigen epitopes". However, these methods are expensive, and need a lot of manual work and material resources. Moreover, they are not suitable for studying complex epitopes. So their applications are limited. The concept of "mimotope" not only provides a clue for analyzing antigen epitope, but also presents a new way for development of vaccine. Mimotope usually represents a polypeptide structure that can mimic an antigen epitope and has a reactionogenicity similar to that of native antigen. When the mimotope is conjugated to a suitable carrier, it may exhibit similar immunogenicity (the native antigen may not comprise an identical or similar sequence or spatial structure). The studies of some antigens that are difficult to be obtained or identified can hardly be conducted, because their antigen epitopes can hardly determined, but this problem may be solved by obtaining their mimotopes. Mimotope provides a clue for analyzing antigen epitope and gives a new way for development of vaccine, moreover, it promotes the researches of conformational epitopes and non-protein antigen epitopes.

One of critical techniques for studying mimotopes is the phage display technique that was established by Dr. Smith in 1985. The construction of phage display peptide library is carried out by the phage displaying technique, which comprises displaying randomly arranged exogenous peptides (usually consisting of 6-15 amino acid residues) on the surface of phage to form a random peptide library having a diversity of more than 10⁷. Through a biological panning procedure of affinity purification, the peptides binding to a target molecule (such as monoclonal antibody, polyclonal antibody, etc.) are screened out from the phage peptide library, then these binding peptides are compared to the native antigen, wherein some have high homology with the native antigen and some are completely different from the native antigen, but all of them have similar antigenicity and immunogenicity with the native antigen, and these binding peptides are called "mimotopes". In comparison with conventional methods of analyzing antigen epitopes, the phage display technique has merits of simple and quick operation and wide applications. It is suitable for studying conformational epitopes, non-protein antigen epitopes and unidentified antigens (Zhong G, et al., J Biol Chem, 1994, 269:24183-24188; Mottic et al., Gene 1994, 146:191-198; Rodriguez L, et al., J Gen Virol 1999, 80(Pt3):727-738).

The screening procedure of using polyclonal antibody or antiserum is difficult in comparison with monoclonal antibody, but has obviously advantages Firstly, the target can be easily obtained, and short time, low cost and simple operation is needed in comparison with the preparation of monoclonal antibody; secondly, mimotopes of several antigen epitopes, can be obtained, thus the screening efficiency is high, facilitating the preparation of composite vaccine with strong immunogenicity. In the present invention, antiserum is purified by immune affinity chromatograph to remove non-specific antibodies and obtain molecules with high specificity, thus specific phage clones can be selected out and the enrichment of non-specific clones can be reduced.

### Invention contents

The object of the present invention is to provide two antigen epitopes and mimotopes of TRAP (Target of RNA III activating protein), a regulatory protein of virulence factor of *staphylococcus aureus.*

Another object of the present invention is to provide the use of said epitopes or polypeptides consistent with said epitopes in amino acid sequences in the manufacture of a vaccine or medicament for combating against *staphylococcus aureus* infections.

For fulfilling the first object of the present invention, TRAP polyclonal antibody is used as target to screen antigen mimotopes of two groups of TRAP from a linear peptide library. As comparing the mimotopes to the TRAP, it is found out that the sequences of said two groups shared common sequences, i.e., two antigen epitopes of TRAP per se: the sequence of amino acids 21-34 and the sequence of amino acids156-167, and their amino acid sequences are as follows:
Antigen epitope 1: corresponding to the sequence of amino acids 21-34 of TRAP: 21 NPTHQLFQFSASDT 34
Antigen epitope 2: corresponding to the sequence of amino acids 156-167 of TRAP: 156 SYFERYLYPIKE 167

The two mimotopes of regulatory protein TRAP of virulence factor in *staphylococcus aureus* of the present invention are polypeptides having the following amino acid sequence:
Antigen mimotope 1: XPXHHQHXTGFT
Antigen mimotope 2: SWFDXXLYPXXX

In the structures of the above antigen epitopes and antigen mimotopes, the capital English letters separately represent an amino acid selected from 21 kinds known native L-type amino acid residues or D-isomers thereof, i.e., A represents alanine residue, R represents arginine residue, N represents asparagine residue, D represents aspartate residue, Q represents glutamine residue, E represents glutamate residue, H represents histidine residue, W represents tryptophane residue, Y represents tyrosine residue, F represents phenylalanine residue, T represents threonine residue, S represents serine residue, L represents leucine residue, G represents glycine residue, P represents proline residue, V represents valine residue, K represents lysine residue, M represents methionine residue, I represents isoleucine residue, X represents any one amino acid residue selected from 21 known native L-type amino acid residues or D-isomers thereof.

In the above epitopes, some amino acids may be replaced with each other according to their similarity, for example, glutamine residue (Q), glutamate residue (E), aspartate residue (D) and asparagine residue (E) may be replaced with each other; tryptophane residue (W), tyrosine residue (Y) and phenylalanine residue (F) may be replaced with each other; lysine residue (K) and arginine residue (R) may be replaced with each other; serine residue (S) and threonine residue (T) may be replaced with each other; alanine residue (A) and glycine residue (G) may be replaced with each other; and leucine residue (L) and methionine residue (M) may be replaced with each other.

The small polypeptide molecules of the present invention may be obtained by chemical synthesis or recombinant expression of genetic engineering.

The experimental results prove that polypeptides having the above structures can specifically compete with TRAP to bind to the polyclonal antibody of TRAP, and inhibit effectively the function of the antibody. Moreover, it exhibit immunogenicity similar to TRAP after conjugated to suitable carriers. The properties of the polypeptides provide a better basis for studying vaccine against *staphylococcus aureus* infections, and thereby facilitate the fulfillment of the second object of the present invention.

The reasons why conventional antibiotic treatment generates drug resistance mainly lie in that, after the administration of an antibiotics, bacteria produce induced enzymes capable of decomposing the effective groups of antibiotics under survival stresses. In the present invention, the polypeptides which the TRAP antigen epitopes and mimotopes thereof correspond to are highly conservative in different *staphylococcus aureus* TRAPs. They can stimulate human body to produce antibodies against TRAP and thereby inhibit the activity of TRAP, so that said polypeptides can be used for preparing a vaccine against *staphylococcus aureus* infections, which can deprive *staphylococcus aureus* of its pathogenicity without killing it. This procedure is suitable for all drug-resistant and non-drug-resistant strains. Thus, a new way is provided for the elimination of drug-resistant *staphylococcus aureus* infections that are common, frequent fatal diseases in clinic.

### Brief description of the drawings

Fig. 1: the electrophoretogram of affinity-purified TRAP polyclonal antibody.
Fig. 2: the gram that phage clone, on which the antigen mimotope sequence 1 was displayed, competed with TRAP for binding to TRAP antibodies, which was detected by a competitive ELISA assay.

The antigen epitopes and mimotopes of the present invention provide a basis for developing vaccines against *staphylococcus aureus* infections, give a new way for the treatment of *staphylococcus aureus* infections, and are widely applicable and highly promising in market.

### Specific modes for carrying out the invention

The present invention is further illustrated in detail by taking the antigen mimotope 1 as an example.

### Example 1: Preparation and purification of polyclonal antibodies against TRAP

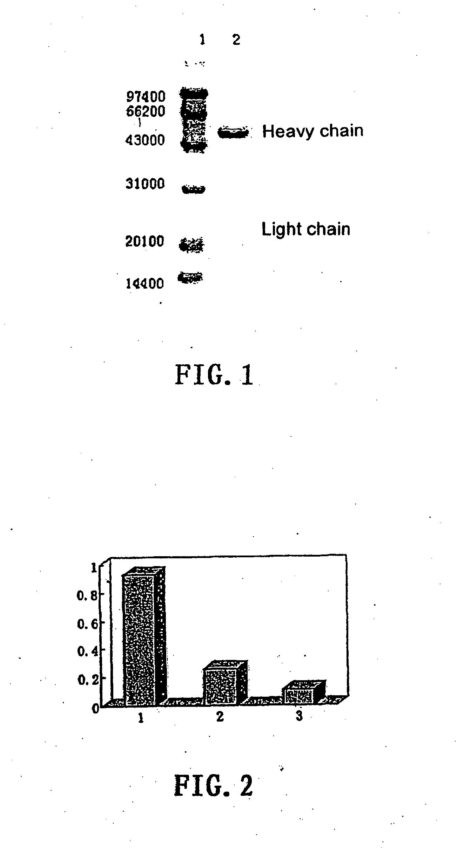

The polyclonal antibodies were produced by immunizing rabbits with purified TRAP. Anti-TRAP IgG was isolated from antiserum by using a TRAP-coupled affinity column. The results of SDS-PAGE electrophoresis indicated that the purified IgG has a purity of >90%, and the results of ELISA indicated that the valence was higher than 1x10⁵ (see Fig. 1, wherein 1 represents a protein standard with low molecular weight, and 2 represents the purified TRAP polyclonal antibodies).

### Example 2: Screening TRAP polyclonal antibodies antigen mimotopes

Firstly, 100µl of the purified TRAP polyclonal antibody IgG was coated on a enzyme-linked immunoassay plate, and placed overnight at 4°C. After the plate was blocked with 2% gelatin for 1 hour, a phage library with peptides of 12 amino acids in length was added, incubated at room temperature for 1 hour, washed with TBST (50mmol/L Tris-HCl, 0.1% TWEEN20, pH7.5) to remove non-specifically binding phages, then specifically binding phages were eluted off with 0.2mmol/L Glycine-HCl pH2.2, and the eluent was neutralized with 1 mmol/L Tris-HCl pH9.0. According to the methods provided by the kit, the titer of the phage in the eluent was determined, and input-output ratio was determined by using the titer of the eluted phage of uncoated target protein as control. In the meantime, the eluted phages binding to anti-TRAP IgG were amplified, and their titers were determined for the next round of screening. After 3 rounds of screening, the input-output ratio increased significantly. The enriched phage clones were identified by ELISA, and 24 positive clones were randomly picked for sequencing. After analysis, the above two groups of polypeptide sequences comprising the antigen mimotope sequence 1 were obtained.

### Example 3: Comparison between the screened antigen mimotope sequence 1 and the expressed TRAP sequence

After analysis of the screened sequence and the original sequence of the expressed TRAP, it was found out that the sequence of the antigen mimotope sequence 1 was similar to the sequence of amino acids 21-34 of TRAP:
Antigen mimotope sequence 1: *NP*LH*HE*HA *TG*W*T*
Primary sequence of TRAP: 21 *NP*T*HQ*LFQF*SA*SD*T* 34

### Example 4: Phage clones of antigen mimotope sequence 1 competed with TRAP for binding to its antibodies

The phage clones of antigen mimotope sequence 1 were selectively amplified, and were detected whether they were capable of competing with TRAP for binding to TRAP polyclonal antibodies. TRAP antibodies were coated, the mixtures of various amounts of TRAP and phages (10⁹) were added, and the enzyme-linked anti-M13 monoclonal antibody was used for detecting whether the phages competed with TRAP for binding to the antibody. The results showed that the phage clones of the antigen mimotope sequence 1 competed with TRAP for binding to the antibodies, and the number of phage clones binding to the antibody gradually decreased with the gradual increase of the amount of TRAP (see Fig. 2, wherein 1 represents 10⁹ of phage clones of antigen mimotope sequence 1, 2 represents 5µg TRAP + 10⁹ of phage clones of antigen mimotope sequence 1, and 3 represents 10µg TRAP + 10⁹ of phage clones of antigen mimotope sequence 1).

### Example 5: Phage clone of antigen mimotope sequence 1 blocked the activity of anti-TRAP polyclonal antibodies

*Staphylococcus aureus* RN6390B was inoculated at a ratio of 1:100. At the same time, the sample was added to CY culture medium and co-cultured for 6 hours. The secretion level of *staphylococcus aureus* exotoxins was determined by MDBK cytotoxic model. The results showed that the anti-TRAP polyclonal antibodies reduced the level of *staphylococcus aureus* exotoxins, while the phage clone of the antigen mimotope sequence 1 inhibited the function of the antibody, i.e., reduced about 25% of the activity of anti-TRAP polyclonal antibodies, indicating that the polypeptides of antigen mimotope sequence 1 blocked the activity of the antibody by specifically binding to the antibody at the portion where the antibody binds to TRAP.

### Example 6: The display of TRAP antigen mimotope sequence 1 on bacterial flagella and the effects of immunizing animals

By utilizing the feature of a high copy number of flagella on bacterial surface (about 20,000 copies per bacterium), the polypeptide corresponding to the TRAP antigen mimotope sequence 1 was displayed in the thioredoxin region of *E. coli* GI826 flagellin, mice were immunized with the recombinant bacteria, and the production of specific antibody was detected after three times of immunization. The results of ELISA and Western Blot showed that the resultant antisera could specifically bind to TRAP, and the titer of the antisera was higher than 5,000, indicating that the obtained mimic peptide induced an immunogenicity similar to that of TRAP after being coupled to a suitable carrier. This provides a better basis for developing a vaccine against *staphylococcus aureus* infections.

## Claims

1. An antigen epitope of TRAP (Target of RNA III activating protein), a regulatory protein of virulence factor in *staphylococcus aureus,* which comprises an amino acid sequence as shown in SEQ ID NO:1 in the sequence listing.

2. An antigen epitope of TRAP (Target of RNA III activating protein), a regulatory protein of virulence factor in *staphylococcus aureus,* which comprises an amino acid sequence as shown in SEQ ID NO:2.

3. A mimotope of TRAP, **characterized in that** it has an amino acid sequence of the following formula:
XPXHHQHXTGFT
wherein X represents any one amino acid selected from 21 known naturally-occurring L-type amino acid residues or D-isomers thereof.

4. A mimotope of TRAP according to claim, **characterized in that** it has an amino acid sequence of the following formula:
SWFDXXLYPXXX
wherein X represents any one amino acid selected from 21 known naturally-occurring L-type amino acid residues or D-isomers thereof.

5. A mimotope of TRAP, **characterized in that** it comprises an amino acid sequence resulting from the replacement of an amino acid in the sequence set forth in claims 1 or 2 on the basis of the similarity between amino acids, said replacement comprising one or more of:
a) replacing glutamine residue (Q) glutamate residue (E), aspartate residue (D) and asparagine residue (E) with each other;
b) replacing tryptophane residue (W), tyrosine residue (Y) and phenylalanine residue (F) with each other;
c) replacing lysine residue (K) and arginine residue (R) with each other;
d) replacing serine residue (S) and threonine residue (T) with each other;
e) replacing alanine residue (A) and glycine residue (G) with each other;
f) replacing leucine residue (L) and methionine residue (M with each other.

6. A mimotope of TRAP, **characterized in that** it comprises an amino acid sequence resulting from the replacement of an amino acids in the sequence set forth in claims 3 or 4 on the basis of the similarity between amino acid, said replacement comprising one or more of:
a) replacing glutamine residue (Q), glutamate residue (E), aspartate residue (D) and asparagine residue (E) with each other;
b) replacing tryptophane residue (W), tyrosine residue (Y) and phenylalanine residue (F) with each other;
c) replacing lysine residue (K) and arginine residue (R) with each other;
d) replacing serine residue (S) and threonine residue (T) with each other;
e) replacing alanine residue (A) and glycine residue (G) with each other;
f) replacing leucine residue (L) and methionine residue (M) with each other.

7. Use of a polypeptide comprising any one of the sequences set forth in claims 1 to 6 in any form for the preparation of a medicament or vaccine against *staphylococcus aureus* infections.

## Patentansprüche

1. Antigen-Epitop von TRAP (Target of RNA III activating protein), einem Regulator-Protein des Virulenzfaktors in *staphylococcus aureus,* umfassend eine Aminosäuresequenz, wie gezeigt in SEQ ID NR:1 in dem Sequenzprotokoll.

2. Antigen-Epitop von TRAP (Target of RNA III activating protein), einem Regulator-Protein des Virulenzfaktors in *staphylococcus aureus,* umfassend eine Aminosäuresequenz, wie gezeigt in SEQ ID NR:2.

3. Mimotop von TRAP, **dadurch gekennzeichnet, dass** es eine Aminosäuresequenz der folgenden Formel hat:
XPXHHQHXTGFT,
wobei X eine beliebige Aminosäure darstellt, ausgewählt aus 21 bekannten, natürlich vorkommenden L-Typ-Aminosäureresten oder D-Isomeren davon.

4. Mimotop von TRAP, **dadurch gekennzeichnet, dass** es eine Aminosäuresequenz der folgenden Formel hat:
SWFDXXLYPXXX,
wobei X eine beliebige Aminosäure darstellt, ausgewählt aus 21 bekannten, natürlich vorkommenden L-Typ-Aminosäureresten oder D-Isomeren davon.

5. Mimotop von TRAP, **dadurch gekennzeichnet, dass** es eine Aminosäuresequenz umfasst, resultierend aus dem Ersetzen einer Aminosäure in der in Anspruch 1 oder 2 dargelegten Sequenz auf der Basis der Ähnlichkeit zwischen Aminosäuren, wobei das Ersetzen eines oder mehr der Folgenden umfasst:
a) Ersetzen von Glutaminrest (Q), Glutamatrest (E), Aspartatrest (D) und Asparaginrest (E) miteinander;
b) Ersetzen von Tryptophanrest (W), Tyrosinrest (Y) und Phenylalaninrest (F) miteinander;
c) Ersetzen von Lysinrest (K) und Argininrest (R) miteinander;
d) Ersetzen von Serinrest (S) und Threoninrest (T) miteinander;
e) Ersetzen von Alaninrest (A) und Glycinrest (G) miteinander;
f) Ersetzen von Leucinrest (L) und Methioninrest (M) miteinander.

6. Mimotop von TRAP, **dadurch gekennzeichnet, dass** es eine Aminosäuresequenz umfasst, resultierend aus dem Ersetzen einer Aminosäure in der in Anspruch 3 oder 4 dargelegten Sequenz auf der Basis der Ähnlichkeit zwischen Aminosäuren, wobei das Ersetzen eines oder mehr der Folgenden umfasst:
a) Ersetzen von Glutaminrest (Q), Glutamatrest (E), Aspartatrest (D) und Asparaginrest (E) miteinander;
b) Ersetzen von Tryptophanrest (W), Tyrosinrest (Y) und Phenylalaninrest (F) miteinander;
c) Ersetzen von Lysinrest (K) und Argininrest (R) miteinander;
d) Ersetzen von Serinrest (S) und Threoninrest (T) miteinander;
e) Ersetzen von Alaninrest (A) und Glycinrest (G) miteinander;
f) Ersetzen von Leucinrest (L) und Methioninrest (M) miteinander.

7. Verwendung eines Polypeptids, umfassend eine beliebige der Sequenzen, dargelegt in den Ansprüchen 1 bis 6 in einer beliebigen Form zur Herstellung eines Arzneimittels oder Impfstoffs gegen Infektionen mit *staphylococcus aureus.*

## Revendications

1. Epitope antigénique de TRAP (cible de protéine activant ARN III), une protéine de régulation de facteur de virulence chez le staphylocoque doré, qui comprend une séquence d'acides aminés telle qu'indiquée dans SEQ ID NO:1 dans la liste des séquences.

2. Epitope antigénique de TRAP (cible de protéine activant ARN III), une protéine de régulation de facteur de virulence chez le staphylocoque doré, qui comprend une séquence d'acides aminés telle qu'indiquée dans SEQ ID NO:2

3. Mimotope de TRAP, **caractérisé en ce qu'**il comporte une séquence d'acides aminés de la formule suivante :
XPXHHQHXTGFT
dans laquelle X représente un acide aminé quelconque choisi parmi 21 résidus d'acides aminés de type L d'origine naturelle connus, ou leurs D-isomères.

4. Mimotope de TRAP, **caractérisé en ce qu'**il comporte une séquence d'acides aminés de la formule suivante :
SWFDXXLYPXXX
dans laquelle X représente un acide aminé quelconque choisi parmi 21 résidus d'acides aminés de type L d'origine naturelle connus, ou leurs D-isomères.

5. Mimotope de TRAP, **caractérisé en ce qu'**il comporte une séquence d'acides aminés résultant de la substitution de certains acides aminés dans la séquence selon les revendications 1 ou 2, sur la base de la similitudes entre des acides aminés, ladite substitution comportant une ou plusieurs des étapes consistant à :
a) remplacer des résidus de glutamine (Q), des résidus de glutamate (E), des résidus d'aspartate (D) et des résidus d'asparagine (E) les uns par les autres ;
b) remplacer des résidus de tryptophane (W), des résidus de tyrosine (Y), et des résidus de phénylalanine (F) les uns par les autres ;
c) remplacer des résidus de lysine (K) et des résidus d'arginine (R) les uns par les autres ;
d) remplacer des résidus de sérine (S) et des résidus de thréonine (T) les uns par les autres ;
e) remplacer des résidus d'alanine (A) et des résidus de glycine (G) les uns par les autres ;
f) remplacer des résidus de leucine (L) et des résidus de méthionine (M) les uns par les autres.

6. Mimotope de TRAP, **caractérisé en ce qu'**il comporte une séquence d'acides aminés résultant de la substitution d'un acide aminé dans la séquence selon les revendications 3 ou 4, sur la base de la similitudes avec un acide aminé, ladite substitution comportant une ou plusieurs des étapes consistant à :
a) remplacer des résidus de glutamine (Q), des résidus de glutamate (E), des résidus d'aspartate (D) et des résidus d'asparagine (E) les uns par les autres ;
b) remplacer des résidus de tryptophane (W), des résidus de tyrosine (Y) et des résidus de phénylalanine (F) les uns par les autres ;
c) remplacer des résidus de lysine (K) et des résidus d'arginine (R) les uns par les autres ;
d) remplacer des résidus de sérine (S) et des résidus de thréonine (T) les uns par les autres ;
e) remplacer des résidus d'alanine (A) et des résidus de glycine (G) les uns par les autres ;
f) remplacer des résidus de leucine (L) et des résidus de méthionine (M) les uns par les autres.

7. Utilisation d'un polypeptide comprenant l'une quelconque des séquences indiquées dans les revendications 1 à 6 sous une forme quelconque, pour la préparation d'un médicament ou d'un vaccin à l'encontre d'infections par le staphylocoque doré.
